# EUROPEAN PATENT SPECIFICATION

(11) **EP 4 133 994 B1**
(45) Date of publication and mention of the grant of the patent: **18.06.2025**
(21) Application number: 20930116.7
(22) Date of filing: 11.06.2020
(51) Int. Cl.: A61B 5/00, A61B 10/02, A61B 5/15, G01N 33/68, G01N 33/53, G01N 33/543

(54) **MIMINALLY-INVASIVE ATOPIC DERMATITIS TEST METHOD USING MICRONEEDLE PATCH AND MINIMALLY-INVASIVE ATOPIC DERMATITIS TEST KIT COMPRISING MICRONEEDLE PATCH**
MIMINAL INVASIVES TESTVERFAHREN FÜR ATOPISCHE DERMATITIS MIT MIKRONADELPFLASTER UND KIT FÜR MINIMALINVASIVE ATOPISCHE DERMATITIS MIT MIKRONADELPFLASTER
PROCÉDÉ DE TEST DE DERMATITE ATOPIQUE MINI-INVASIF UTILISANT UN TIMBRE À MICRO-AIGUILLES ET KIT DE TEST DE DERMATITE ATOPIQUE MINI-INVASIF COMPRENANT UN TIMBRE À MICRO-AIGUILLES

(30) Priority: 07.04.2020 KR 20200041957
(43) Date of publication of application: 15.02.2023
(73) Proprietor: Raphas Co., Ltd., Seoul 07793 (KR)
(72) Inventor: JEONG, Do Hyeon, Seoul 04091 (KR); KIM, Jung Dong, Incheon 22704 (KR); LEE, Keun Ho, Goyang-si, Gyeonggi-do 10508 (KR); LEE, Kwang Hoon, Seoul 07333 (KR)
(74) Representative: M. Zardi & Co S.A.
(86) International application number: PCT/KR2020/007612
(87) International publication number: WO 2021/206218

(56) References cited:
- EP-A1- 3 498 175
- WO-A1-2008/096732
- JP-A- 2014 156 433
- KR-A- 20170 142 127
- KR-A- 20190 132 291
- KR-B1- 101 878 414
- PAVEL ANA B ET AL: "The proteomic skin profile of moderate-to-severe atopic dermatitis patients shows an inflammatory signature", JOURNAL OF THE AMERICAN ACADEMY OF DERMATOLOGY, MOSBY, INC, US, vol. 82, no. 3, 25 October 2019 (2019-10-25), pages 690 - 699, XP086041870, ISSN: 0190-9622, [retrieved on 20191025], DOI: 10.1016/J.JAAD.2019.10.039
- GUTTMAN-YASSKY EMMA ET AL: "Use of Tape Strips to Detect Immune and Barrier Abnormalities in the Skin of Children With Early-Onset Atopic Dermatitis", JAMA DERMATOLOGY, vol. 155, no. 12, 1 December 2019 (2019-12-01), US, pages 1358, XP055912252, ISSN: 2168-6068, DOI: 10.1001/jamadermatol.2019.2983

## Description

### Technical Field

The present invention relates to a minimally invasive method of diagnosing atopic dermatitis using a microneedle patch and a minimally invasive kit for diagnosing atopic dermatitis including a microneedle patch.

### Background Art

Numerous drugs and physiologically active substances have been developed for the treatment of diseases. However, in the delivery of drugs and physiologically active substances into the body, problems associated with passage through biological barriers (e.g., skin, oral mucosa, brain-vascular barrier, etc.) and with the efficiency of drug delivery still remain as problems to be solved.

Drugs and physiologically active substances are generally administered orally in tablet or capsule form, but many drugs cannot be effectively delivered by this administration method because they are digested or absorbed in the gastrointestinal tract or lost due to a liver mechanism. Furthermore, some drugs cannot effectively diffuse across the intestinal mucosa. For example, in the case of patients who have to take medications at specific time intervals or critical patients who cannot take medications, patient compliance also becomes a problem.

Another common technique for the delivery of drugs and bioactive substances is the use of a conventional injection needle. This method is more effective than oral administration, but has problems in that it causes pain at the injection site, local damage to the skin, bleeding, and disease infection at the injection site.

In order to solve the problems of oral administration and subcutaneous injection, transdermal administration using a patch is used. The transdermal administration using a patch has fewer side effects and high patient compliance is easy to maintain a constant blood drug concentration.

In order to solve the above problems, various microstructures including microneedles have been developed. As materials for the microneedle, metals and various polymer materials have been used. In recent years, biodegradable polymer materials have attracted attention as materials for the microneedles. The patent application EP 3 498 175 A1 discloses a micro-needle patch based on hyaluronic acid for diagnosing allergic diseases, where the micro-needles are constructed to inject an allergen into the patient.

A representative method for fabricating a microstructure formed of a biodegradable material is a method using a mold. A mold having an intaglio corresponding to the shape of a microstructure to be formed is first fabricated by applying a semiconductor fabrication process, and a microstructure material is poured into such a mold, solidified and then separated from the mold, thereby forming a microstructure, that is, a microneedle.

The present applicant has a number of domestic and foreign patents related to a method of fabricating a microstructure using a droplet-born air blowing process, as a new fabrication method that may replace the mold-based microstructure fabrication method. The droplet-born air blowing process will be described in brief below. The droplet-born air blowing process includes: spotting a viscous biocompatible polymer substance either on the bottom layer of a patch placed on one substrate or on the bottom layer of the patch before being coupled to the patch; spotting the same biocompatible polymer substance either on the bottom layer of a patch placed on another substrate or on the bottom layer of the patch before being coupled to the patch (or omitting this operation); inverting the other substrate upside down; approaching the inverted other substrate toward the one substrate so that the spotted biocompatible polymer substance on the inverted other substrate is brought into contact with the spotted biocompatible polymer substance on the one substrate; spacing the two substrates apart from each other to stretch (elongate) the viscous biocompatible polymer substances which have been brought into contact with each other; performing air blowing after the stretching operation to fix the stretched state of the biocompatible polymer substance; and cutting a middle portion of the stretched biocompatible polymer substance so that the same microstructure is formed on each of the two substrates.

In the above, the technical meaning of the microstructure as a means for delivering drugs and bioactive substances, the method of fabricating the microstructure, and the like have been described in brief. The present applicant has reached the present invention and proposes to use the microstructure for a new purpose rather than the delivery of drugs and physiologically active substances. In other words, a patch including the microstructure, that is, a microneedle, is used for minimally invasive diagnosis of atopic dermatitis.

Atopic dermatitis is an inflammatory skin disease accompanied by itching, and is the most representative allergic disease along with allergic rhinitis and bronchial asthma. Atopic dermatitis occurs mainly in infants and children with a prevalence of 10 to 30%, but in recent years, atopic dermatitis in adults has increased. In the main pathological mechanism of atopic dermatitis, abnormalities in the skin barrier, immunological abnormalities, genetic predispositions, environmental predispositions, etc. are involved. Abnormalities in the skin barrier are caused by a decrease in the synthesis of numerous biological factors (such as filaggrin), which are involved in the differentiation of keratinocytes, and ceramide, which is an important component of skin barrier lipids, and cause skin dryness and itching. Immunological abnormalities are caused by the complex action of innate and adaptive immune responses caused by cells such as dendritic cells, T cells, and other innate immune cells (mast cells, eosinophils, basophils, neutrophils), which secrete various cytokines, such as TSLP, IL-4 and IL-13, and inflammatory substances such as chemokines. Thereamong, the most key cytokines in pathogenesis are IL-4 and IL-13. Recently, many biologic drugs targeting these markers have been developed and widely used as new therapeutic agents for intractable atopic dermatitis and exhibit very excellent effects.

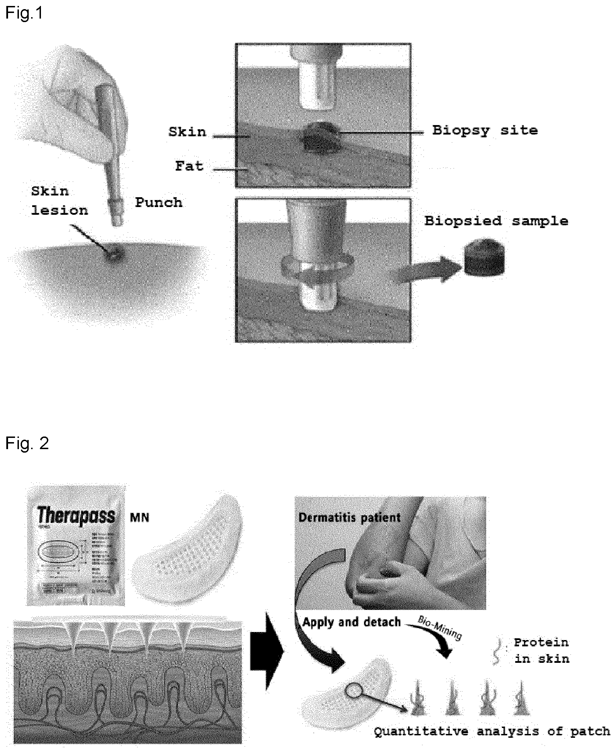

Observation of changes in numerous biological substances related to the pathogenesis of atopic dermatitis as described above is associated with the development of new therapeutic drugs. In order to discover a new pathogenesis of atopic dermatitis, determine the effect of drugs on the treatment of atopic dermatitis, predict the prognosis of atopic dermatitis, and discover a new therapeutic agent, a method of biopsying the lesion site of atopic dermatitis and analyzing atopic dermatitis-related biological factors in the tissue is most commonly used. FIG. 1 shows such a biopsy. However, the biopsy requires the expert skill of an operating surgeon. In addition, the biopsy has disadvantages in that it causes repulsion due to pain caused to a patient and leaves a scar at the biopsy site after the biopsy. For these reasons, it is very difficult to detect these factors one by one in the human body, and an obstacle to the development of new technologies or new drugs occurs. In order to overcome these disadvantages, a non-invasive stripping method using a tape has been proposed, in which a tape is attached to and detached from a lesion site and a skin sample attached to the tape is analyzed. However, this stripping method has a disadvantage in that the substance attached to the tape is limited to the stratum corneum. Meanwhile, since atopic dermatitis is a skin disease caused by immune system abnormalities in the stratum corneum, epidermis, and dermis, it is important to sample tissue from both epidermis and dermis, like a biopsy. Thus, the tape stripping method may be used as an auxiliary method for new drug development for atopic dermatitis or researches on the pathogenesis of atopic dermatitis, but it is difficult for the tape stripping method to obtain a complete result. Accordingly, the present inventors obtained skin samples using skin biopsy, tape stripping and various types of microstructure patches, and examined whether a skin sample capable of replacing a skin biopsy sample is obtained through stripping using a tape or a microstructure patch, in order to confirm the usefulness of a sampling method capable of replacing skin biopsy.

A total of three types of microstructure patches were used in this study and include: a microstructure-free blank patch as a control for a microstructure patch; a hollow microstructure patch; and a soluble microstructure patch. The blank patch as a control is generally composed of only a hydrocolloid patch that is mainly used for wound healing. The hollow microstructure patch has advantages that it is possible to minimize pain and infection risk through a hole having a micro-sized cross-sectional area in the microstructure, and at the same time, drug delivery is possible. In the soluble microstructure patch, a microneedle including a pharmaceutical-grade hyaluronic acid component is formed on the hydrocolloid patch. Methods for fabricating microstructure patches include the above-described mold-based method and droplet-born air blowing method, and any fabrication method may be used as long as a microneedle including a hyaluronic acid component is formed on a patch. When these microstructure patches are applied to the skin, as shown in FIG. 2, the microstructures penetrate the stratum corneum and enter the skin. The present inventors attached the patch to the skin for a sufficient time for various factors in the epidermis and dermis to be adsorbed onto the microstructures that have entered the skin, and then attempted to obtain a skin sample from the detached patch. Next, the present inventors performed comparative analysis between tissue biopsy, tape stripping and three types of microstructure patch methods in order to propose a novel minimally invasive method for diagnosing atopic dermatitis that may be used instead of tissue biopsy to detect important biomarkers involved in pathogenesis or measure the expression levels of the biomarkers.

### DISCLOSURE

### Technical Problem

An object of the present invention is to solve the problems of the conventional method of diagnosing atopic dermatitis by tissue biopsy or tape stripping.

Conventional tissue biopsy has disadvantages in that it requires the expert skill of an operating surgeon, causes repulsion due to pain caused to a patient, and leaves a scar at the biopsy site after the biopsy.

Conventional tape stripping has a disadvantage in that the substances attached to the tape are limited to the stratum corneum, and thus may be used only as an auxiliary diagnostic method and does not provide complete results.

An object of the present invention is to provide a novel minimally invasive method for diagnosing atopic dermatitis and a novel minimally invasive kit for diagnosing atopic dermatitis, which cause little pain, do not leave a scar, and may increase patient compliance, compared to conventional tissue biopsy, and at the same time, may increase the reliability of analysis results compared to conventional tape stripping by sampling skin biomarkers from the stratum corneum.

### Technical Solution

A minimally invasive method for diagnosing atopic dermatitis according to one embodiment of the present invention includes steps of: applying, to the skin of a subject, a microneedle patch including a plurality of microneedles, which are formed of biodegradable polymer hyaluronic acid and have a solid structure, and a bottom layer on which the plurality of microneedles are formed; maintaining the microneedle patch for a predetermined time in a state in which the microneedle patch is attached to the skin of the subject; separating the microneedle patch from the skin of the subject after the predetermined time and putting the separated microneedle patch into quantitative testing; reading the amounts of interleukin-4 and interleukin-13, adsorbed onto the microneedle surface of the microneedle patch, in the quantitative testing; and measuring the activity of atopic dermatitis based on the read amounts of interleukin-4 and interleukin-13.

A minimally invasive kit for diagnosing atopic dermatitis according to one embodiment of the present invention includes: a device configured to quantitatively analyze the amount of protein extracted from the skin of a subject; and a microneedle patch including a plurality of microneedles, which are formed of biodegradable polymer hyaluronic acid and have a solid structure, and a bottom layer on which the plurality of microneedles are formed. The microneedle patch is applied to the skin of the subject, maintained for a predetermined time, and then separated from the skin, and in the separated state, the proteins from the subject, adsorbed onto surfaces of the microneedles of the microneedle patch, are quantitatively analyzed by the device. The device reads the amounts of interleukin-4 and interleukin-13 adsorbed onto surfaces of the microneedles of the microneedle patch. The activity of atopic dermatitis is evaluated based on the read amounts of interleukin-4 and interleukin-13.

In addition, an additional configuration may be further provided in the minimally invasive method for diagnosing atopic dermatitis or the minimally invasive kit for diagnosing atopic dermatitis according to the present invention.

### Advantageous Effects

According to the present invention, it is possible to solve the problems of the conventional method of diagnosing atopic dermatitis by tissue biopsy or tape stripping.

Conventional tissue biopsy has disadvantages in that it requires the expert skill of an operating surgeon, causes repulsion due to pain caused to a patient, and leaves a scar at the biopsy site after the biopsy.

Conventional tape stripping has a disadvantage in that the substances attached to the tape are limited to the stratum corneum, and thus may be used only as an auxiliary diagnostic method and does not provide complete results.

According to the present invention, it is possible to provide a novel minimally invasive method for diagnosing atopic dermatitis and a novel minimally invasive kit for diagnosing atopic dermatitis, which cause little pain, do not leave a scar, and may increase patient compliance, compared to conventional tissue biopsy, and at the same time, may increase the reliability of analysis results compared to conventional tape stripping by sampling skin biomarkers from the stratum corneum.

### Brief Description of Drawings

FIG. 1 shows a skin biopsy method according to a conventional art.
FIG. 2 conceptually illustrates a skin biopsy method according to the present invention.
FIG. 3 shows four controls in an experiment (hereinafter referred to as basic experiment 1) conducted by the present inventors on a minimally invasive skin biopsy method closely related to the concept of the present invention prior to a clinical trial related to a diagnostic method for atopic dermatitis. Specifically, FIG. 3 shows 1) a blank patch in which microneedles are not formed, 2) metallic solid microneedles, 3) a low-molecular-weight hyaluronic acid microneedle patch, and 4) a high-molecular-weight hyaluronic acid microneedle patch.
FIG. 4 is a table showing the results of conducting a pro-collagen 1 detection test for a patient group aged 30 to 39 years and a patient group aged 60 to 69 using the four controls of basic experiment 1.
FIG. 5 is a table showing the results of measuring changes in length of microneedles before and after the low-molecular-weight hyaluronic acid microneedle patch and the high-molecular-weight hyaluronic acid microneedle patch among the four controls of basic experiment 1 are applied to skin biopsy.
FIG. 6 is a table showing the results of another basic experiment (hereinafter referred to as "basic experiment 2"). Controls used here are 1) a blank patch in which microneedles are not formed, 2) a low-molecular-weight hyaluronic acid microneedle patch, and 3) a high-molecular-weight hyaluronic acid microneedle patch. Each of the controls was applied to the skins of a patient group aged 20 to 29 years and a patient group aged 60 to 69 years for 5 seconds and 60 seconds and then detached, and the amount of pro-collagen 1 detected was measured.
FIG. 7 shows the results of measuring the amount of detected pro-collagen 1 after the controls according to basic experiment 2 were applied to the skins of a patient group aged 20 to 29 years and a patient group aged 60 to 69 years for 10 seconds and 30 seconds and then detached.
FIG. 8 is a table showing the results of still another basic experiment (hereinafter referred to as "basic experiment 3"). Controls used here are 1) a low-molecular-weight chitosan microneedle patch, and 2) a high-molecular-weight chitosan microneedle patch. Each of the controls was applied to the skin for 5 seconds, 60 seconds and 300 seconds and then detached, and the amount of pro-collagen 1 detected was measured.
FIG. 9 shows photographs of three types of patches used in a clinical test to verify the effect of the present invention.
FIG. 10 is a table showing a clinical trial schedule.
FIG. 11 is a table summarizing information on clinical trial subjects.
FIG. 12 is a table showing the amounts of protein attached to four types of means for obtaining skin samples: tape, blank patch, HA microneedle, and hollow needle.
FIGS. 13 and 14 are graphs showing the results of analyzing the expression level of each of IL-13 and IFN-gamma using a tape and a microneedle.
FIG. 15 is a table showing the results of ELISA assay performed to measure the expression of IL-4 in skin samples obtained from clinical trial subjects by HA microneedles.
FIG. 16 is a table showing the results of ELISA assay performed to measure the expression of IL-13 in skin samples obtained from clinical trial subjects by HA microneedles.
FIG. 17 is a table showing the results of ELISA assay performed to measure the expression of IFN-gamma in skin samples obtained from clinical trial subjects by HA microneedles.
FIG. 18 shows the results of statistical analysis (SPSS) performed to analyze the correlation between the expression of IL-4 in skin samples obtained from clinical trial subjects by HA microneedles and the clinical course of atopic dermatitis.
FIG. 19 shows the results of statistical analysis (SPSS) performed to analyze the correlation between the expression of IL-13 in skin samples obtained from clinical trial subjects by HA microneedles and the clinical course of atopic dermatitis.
FIG. 20 shows the results of statistical analysis (SPSS) performed to analyze the correlation between the expression of IFN-gamma in skin samples obtained from clinical trial subjects by HA microneedles and the clinical course of atopic dermatitis.

### Mode for Invention

Reference is made to the accompanying drawings, which show, by way of illustration, specific embodiments in which the present invention may be practiced. These embodiments are described in sufficient detail to enable those skilled in the art to practice the present invention. It should be understood that various embodiments of the present invention are different but need not be mutually exclusive. The following detailed description is not to be taken in a limiting sense, and the scope of the present invention should be construed as covering the scope of the claims and all equivalents thereto.

Hereinafter, various preferred embodiments of the present invention will be described in detail with reference to the accompanying drawings in order to enable those of ordinary skill in the art to easily practice the present invention.

### Results of basic experimental results on minimally invasive skin biopsy method

FIG. 2 conceptually illustrates a skin testing method according to the present invention.

The upper left of FIG. 2 shows a commercially available microneedle patch with the trade name "Therapass". This product is a high-molecular-weight hyaluronic acid microneedle patch with a molecular weight of 1,000 kDa, which is marketed and sold by the applicant company. The lower left of FIG. 2 shows a state in which the microneedle patch has been applied to the skin, more specifically, a state in which the microneedles of the patch have penetrated the dermal layer of the skin. The right part of FIG. 2 conceptually illustrates a representative technical idea of the present invention in which a microneedle patch is applied to a dermatitis patient and then a biopsy is performed using the skin protein attached to the microneedles of the patch. The term "bio-mining" indicated in FIG. 2 is used to mean extracting proteins in the skin of a subject for biopsy.

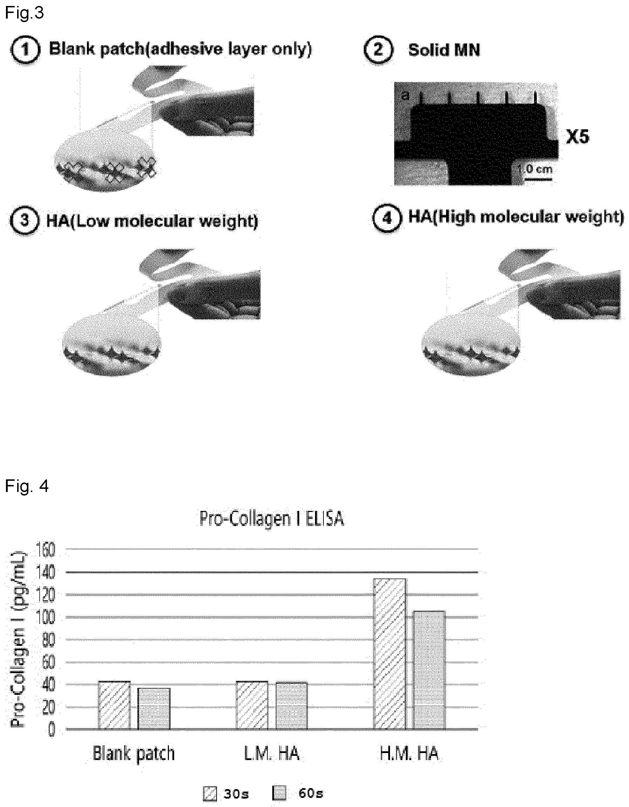

FIG. 3 shows four controls in basic experiment 1 conducted by the present inventors on a minimally invasive skin biopsy method closely related to the concept of the present invention prior to a clinical trial related to a diagnostic method for atopic dermatitis. Among these four controls, the first control is a blank patch in which microneedles are not formed. The second control is metallic solid microneedles. The third control is a low-molecular-weight hyaluronic acid microneedle patch. The molecular weight of hyaluronic acid used in the low-molecular-weight hyaluronic acid microneedle patch is110 kDa. The fourth control is a high-molecular-weight hyaluronic acid microneedle patch. The molecular weight of hyaluronic acid used in the high-molecular-weight hyaluronic acid microneedle patch is 1,000 kDa.

FIG. 4 is a table showing the results of conducting a pro-collagen 1 detection test for a patient group aged 30 to 39 years and a patient group aged 60 to 69 using the above-described four controls.

The x-axis of FIG. 4 shows a blank patch, a low-molecular-weight hyaluronic acid patch, and a high-molecular-weight hyaluronic acid patch. The y-axis shows the mass per unit volume of detected pro-collagen 1 in units of picograms per milliliter. Among the four controls, the metallic solid microneedles are not shown. The reason is that protein quantification itself was not performed at all. "ELISA" shown in FIG. 4 refers to a method that is used in modern biotechnology to quantitatively measure the intensity and amount of an antigen-antibody reaction by labeling an antibody or antigen with an enzyme and measuring the activity of the enzyme. This method was used throughout the experiments conducted in the present invention.

What should be looked at carefully in the table of FIG. 4 is whether the detection result is substantially different from that obtained by the blank patch that is the first control. This is because the blank patch is a conventional art that existed prior to the filing of the present invention, and if the detection result is not substantially different from that obtained by the blank patch, it means that the present invention does not contribute to the improvement of the detection effect. Referring to the results in FIG. 4, the amount of pro-collagen 1 detected in the low-molecular-weight (110 kDa) hyaluronic acid patch was not substantially different from that in the blank patch. However, it can be seen that the amount of pro-collagen 1 detected in the high-molecular-weight (1,000 kDa) hyaluronic acid patch significantly increased compared to those in the blank patch and the low-molecular-weight hyaluronic acid patch.

The present inventors continued experiments and analyzed the cause of how this remarkable difference in the results appeared depending on the molecular weight of hyaluronic acid, which is a component of the microneedle patch, and found the cause in the rate of dissolution into the skin depending on the difference in the molecular weight. The microneedle component of a currently commercialized microneedle patch is generally a biocompatible and biodegradable polymer material. "Biocompatible material" refers to a material that is not toxic to the human body and is chemically inert. In addition, "biodegradable material" refers to a material that may be degraded *in vivo* by body fluids, enzymes or microorganisms. In addition, it is known that the dissolution rate of biodegradable materials *in vivo* tends to increase as the molecular weight becomes smaller, and tends to decrease as the molecular weight becomes higher. Meanwhile, the following materials are known as biocompatible polymers:

Hyaluronic acid (HA), gelatin, chitosan, collagen, alginic acid, pectin, carrageenan, chondroitin (sulfate), dextran (sulfate), polylysine, carboxymethyl chitin, fibrin, agarose, pullulan, cellulose, polyvinylpyrrolidone (PVP), polyethylene glycol (PEG), polyvinyl alcohol (PVA), hydroxypropyl cellulose (HPC), hydroxyethyl cellulose (HEC), hydroxypropyl methyl cellulose (HPMC), sodium carboxymethylcellulose, polyalcohol, gum Arabic, alginate, cyclodextrin, dextrin, glucose, fructose, starch, trehalose, glucose, maltose, lactose, lactulose, fructose, turanose, melitose, melezitose, dextran, sorbitol, xylitol, palatinit, polylactic acid, polyglycolic acid, polyethylene oxide, polyacrylic acid, polyacrylamide, polymethacrylic acid, polymaleic acid, a poly(ethyleneglycol)/polyester) copolymer, chitosan/glycerol phosphate, polyphosphazene, polycaprolactone, polycarbonate, poly(ethylene glycol)/poly(propylene glycol), polycyanoacrylate, polyorthoester, poly(N-(2-hydroxyethyl)methacrylamide-lactate, poly(propylene phosphate), and the like.

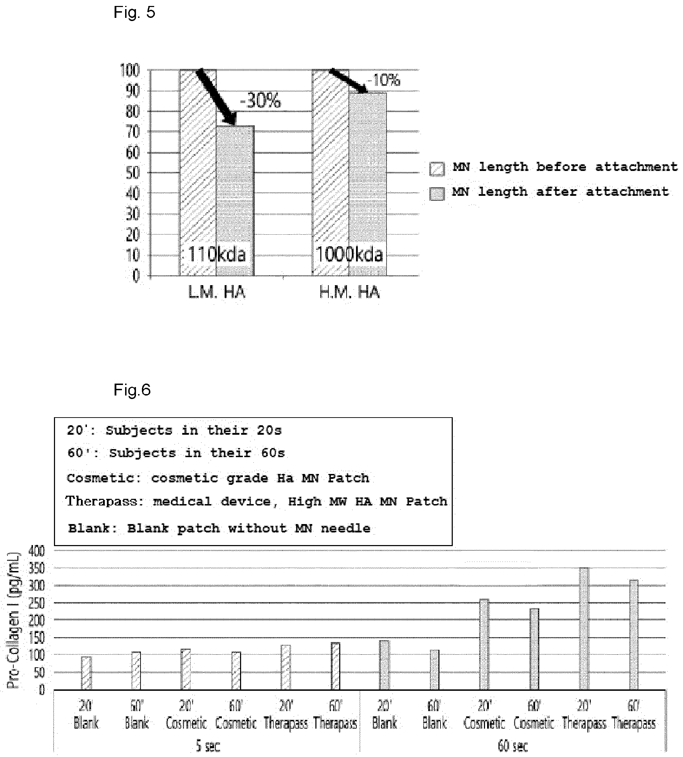

When a microneedle patch is attached to the skin, protein may be extracted after it adheres to the biocompatible microneedle structure in a process in which the microneedles of the patch enter the dermal layer of the skin and in a process in which the microneedles are maintained after the entry. However, the low-molecular-weight hyaluronic acid microneedle has a relatively high dissolution rate even if protein *in vivo* adheres to the surface of the microneedle, and thus when the microneedle is attached to the skin for a long time, the protein attached to the surface may be lost while the microneedle itself is dissolved. It is believed that the experimental results in FIG. 4 were obtained for this reason. The results supporting this belief are shown in FIG. 6.

FIG. 5 is a table showing the results of measuring changes in length of microneedles before and after the low-molecular-weight hyaluronic acid microneedle patch and the high-molecular-weight hyaluronic acid microneedle patch among the above-described four controls are applied to skin biopsy.

Referring to the results in FIG. 5, the microneedles of the 110 kDa low-molecular-weight hyaluronic acid microneedle patch showed a decrease in length of approximately 30% during their attachment to the human skin. On the other hand, the microneedles of the 1,000 kDa high-molecular-weight hyaluronic acid microneedle patch showed a decrease in length of only about 10% during their attachment to the skin. These experimental results support that the low-molecular-weight hyaluronic acid microneedle has a relatively high dissolution rate even if protein *in vivo* adheres to the surface thereof, and thus when the microneedle is attached to the skin for a long time, the protein attached to the surface may be lost while the microneedle itself is dissolved, and this protein loss is the cause of the experimental results shown in FIG. 4. In experiment 1 shown in FIGS. 4 and 5, each control was attached to the skin for 5 minutes. Accordingly, the present inventors have found the need to perform experiments for different lengths of time by reducing the time of attachment to the skin to less than 5 minutes, and planned an experiment (experiment 2) different from the above-described basic experiment (experiment 1).

FIG. 6 is a table showing the results of basic experiment 2 different from the above-described basic experiment 1. Controls used here are 1) a blank patch in which microneedles are not formed, 2) a low-molecular-weight hyaluronic acid microneedle patch, and 3) a high-molecular-weight hyaluronic acid microneedle patch. Each of the controls was applied to the skins of a patient group aged 20 to 29 years and a patient group aged 60 to 69 years for 5 seconds and 60 seconds and then detached, and the amount of pro-collagen 1 detected was measured. The molecular weight of the low-molecular-weight hyaluronic acid and the molecular weight of the high-molecular-weight hyaluronic acid are the same as those in basic experiment 1. As the low-molecular-weight hyaluronic acid microneedle patch, a microneedle patch for cosmetic use sold by the applicant was used, and as the high-molecular-weight hyaluronic acid microneedle patch, a microneedle patch for medical device use (trade name: "Therapass") sold by the applicant was used. Since the experimental results of basic experiment 1 indicated that the bioprotein extraction performance of the metallic solid microneedle was significantly insufficient, the metallic solid microneedle was not used as a control in experiment 2.

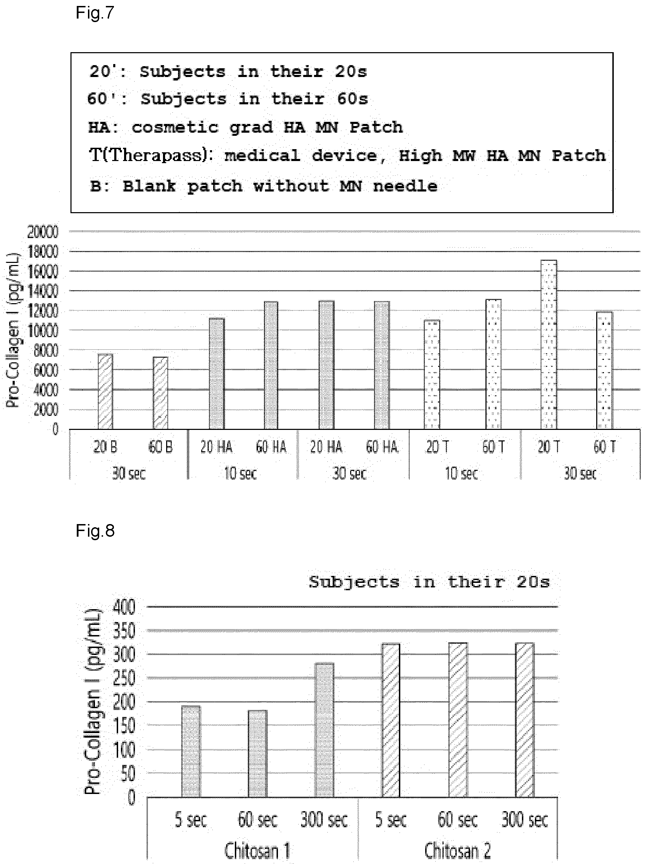

The following facts can be seen from the experimental results shown in FIG. 6. First, when the attachment time is 5 seconds, a significant improvement in the results obtained by the microneedle patch compared to those obtained by the blank patch cannot be expected. Second, when the attachment time is 60 seconds, the microneedle patches (low-molecular-weight and high-molecular-weight) show significantly superior bioprotein extraction performance compared to the blank patch. Third, even when the attachment time is 60 seconds, the bioprotein extraction performance of the high-molecular-weight microneedle patch is superior to the bioprotein extraction performance of the low-molecular-weight microneedle patch. The present inventors additionally conducted the experiment shown in FIG. 7 to obtain additional results for an attachment time longer than 5 seconds and shorter than 60 seconds, based on the experimental results shown in FIG. 6.

FIG. 7 shows the results of measuring the amount of detected pro-collagen 1 after the controls according to basic experiment 2 were applied to the skins of a patient group aged 20 to 29 years and a patient group aged 60 to 69 years for 10 seconds and 30 seconds and then detached.

In the case of the blank patch, there was no significant change in the results either when the attachment time was 10 seconds or when the attachment time was 30 seconds, and the table in FIG. 7 shows only the results obtained when the attachment time was 30 seconds. The facts that can be seen from the results shown in FIG. 7 are as follows. First, even when the attachment time is 10 seconds, it can be confirmed that the bioprotein extraction performance of the microneedle patches (low-molecular-weight and high-molecular-weight) is significantly improved compared to that of the blank patch. Second, when the attachment time is 10 seconds, the protein extraction performance of the low-molecular-weight microneedle patch is substantially the same as that of the high-molecular-weight microneedle patch. Third, when the attachment time is 30 seconds, the protein extraction performance of the high-molecular-weight microneedle patch is remarkably superior to the protein extraction performance of the low-molecular-weight microneedle patch.

It is evaluated that the technical significance of the experiment shown in FIG. 7 is that the microneedle patch can exhibit significant biopsy performance when an attachment time of 10 seconds or more is maintained, and that the low-molecular-weight microneedle patch shows substantially the same biopsy performance as the high-molecular-weight microneedle patch at an attachment time of about 10 seconds, which is before the time for the low molecular weight microneedle to dissolve into the skin has elapsed.

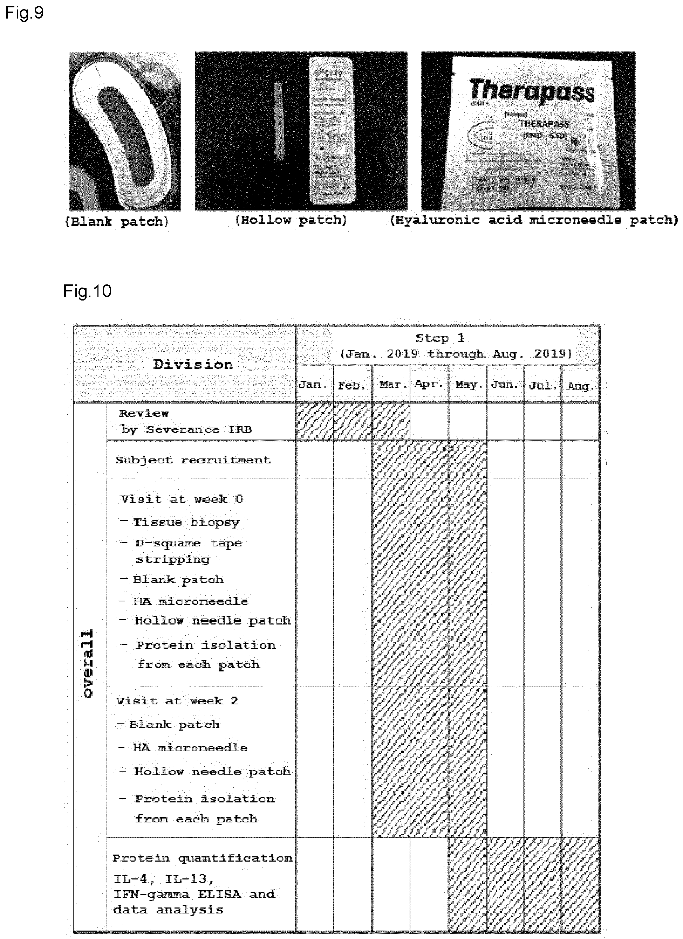

Finally, the present inventors conducted the experiment (basic experiment 3) shown in FIG. 9 using a microneedle patch made of chitosan in order to examine whether a microneedle patch made of a biocompatible polymer material other than hyaluronic acid would be suitable for use in biopsy. Chitosan is a biocompatible high-molecular-weight material, like hyaluronic acid, but is not biodegradable. Chitosan is a material obtained by processing chitin contained in crustaceans so as to be easily absorbed in the human body, and is known to have the effects of inhibiting senescence and strengthening immunity by activating degenerated cells. Chitosan is widely used as a cosmetic ingredient because its effect on skin cell activation has been proven, and is also used as a component of a microneedle patch for skin care purposes.

FIG. 8 is a table showing the results of basic experiment 3". Controls used here are 1) a low-molecular-weight chitosan microneedle patch, and 2) a high-molecular-weight chitosan microneedle patch. Each of the controls was applied to the skin for 5 seconds, 60 seconds and 300 seconds and then detached, and the amount of pro-collagen 1 detected was measured.

In the experiment shown in FIG. 8, the molecular weight of low-molecular-weight chitosan 1 is 50 to 100 kDa. The molecular weight of high-molecular-weight chitosan 2 is 250 to 350 kDa. For a patient group aged 20 to 29 years, each chitosan microneedle patch was attached for 5 seconds, 1 minute, and 5 minutes, and then the expression of pro-collagen I was analyzed. From the experimental results of experiment 3 shown in FIG. 8, the present inventors confirmed that chitosan, which is a biocompatible polymer material, could exert a protein extraction function for biopsy, like hyaluronic acid. It can be seen that various other biocompatible polymer materials other than hyaluronic acid and chitosan exemplified in the present specification may also be considered sufficient candidates for microneedle patch materials for minimally invasive skin biopsy.

### Results of study on novel diagnostic method for atopic dermatitis (conduction of clinical trial)

FIG. 9 shows photographs of three types of patches used in a clinical test to verify the effect of the present invention.

The blank patch on the left side of FIG. 9 is composed of only a hydrocolloid material patch, and a needle structure is not formed thereon. The hollow patch in the middle of FIG. 9 is a microneedle commercially available from INCYTO, and has a structure in which a hole having a micro-sized cross-sectional area is formed in the center of an elongated metal needle. As the hyaluronic acid (HA) microneedle patch on the right of FIG. 9, a commercially available microneedle patch (trade name: "Therapass") is shown. This product is a high-molecular-weight hyaluronic acid microneedle patch with a molecular weight of 1,000 kDa, which is marketed and sold by the applicant company.

This clinical trial was conducted on 20 patients with atopic dermatitis (forearm). The study period was 5 months after IRB approval. The study method is as follows.

### 1) Week 0

- Biopsy: 3 mm biopsy
- Tape stripping: 15 strippings with D-Squame disk tape
- Protein extraction: protein extraction from tapes or microneedle patches using SDS

### 2) Week 2

- Testing using 3 types of microneedle patches: attachment of 3 types of microneedle patches for 5 minutes, followed by detachment
- Protein extraction: protein extraction from tapes or microneedle patches using SDS

In this way, testing on each subject was conducted at week 0 and week 2, and the clinical usefulness of the testing method was evaluated by comparing the test result with the clinical course. In this evaluation, protein quantification was performed by BCA assay, and IL-4, IL-13, and IFN-gamma were quantified by performing ELISA assay. SPSS was used as a statistical analysis technique.

FIG. 10 is a table showing a clinical trial schedule. As described above, a clinical trial was conducted for 5 months after IRB. After test subjects were recruited, tissue biopsy, D-squame tape stripping, blank patch, HA microneedle, and hollow needle patch were applied to the subjects upon visit at week 0, and proteins were isolated from each of the detection means. Finally, the amounts of isolated proteins were quantified, and ELISA was performed for IL-4, IL-13, IFN-gamma, followed by data analysis.

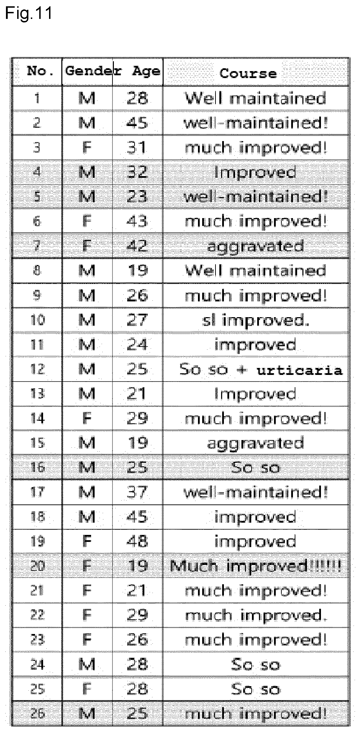

FIG. 11 is a table summarizing information on clinical trial subjects. The table in FIG. 11 shows the gender, age, and post-2-week symptom course of each subject who participated in this clinical trial (the expression "sl improved" for the course of patient No. 10 in the table denotes slightly improved, that is, a slightly improved course). Except for fail, a total of 20 subjects were enrolled and the study was conducted. Subjects No. 4, No. 5, No. 7, No. 16, No. 20, and No. 26, indicated to be distinguishable from other subjects in the table in FIG. 11, refused to second patch attachment/detachment, and thus were excluded from the subjects.

At week 0, skin samples were obtained using forearm biopsy, tape stripping, and three types of microneedle patches, and at week 2, skin samples were obtained using three types of microneedle patches. The obtained samples were placed in 60-mm dishes and immediately protein was extracted therefrom using an SDS solution in the laboratory and stored at -80°C.

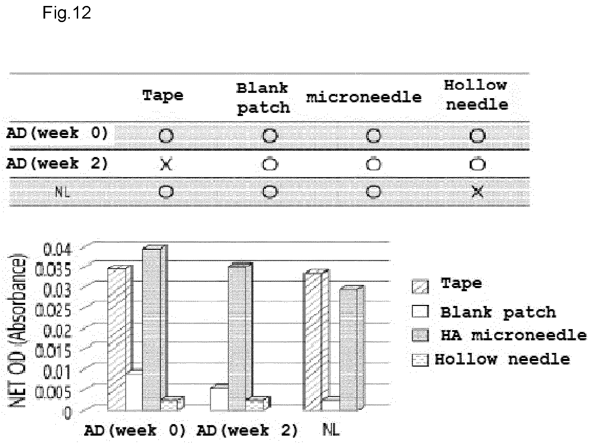

FIG. 12 is a table showing the amounts of protein attached to four types of means for obtaining skin samples: tape, blank patch, HA microneedle, and hollow needle. The obtained skin samples were quantified for protein by BCA assay. At week 0, the tape, blank patch, hyaluronic acid microneedle, and hollow needle were all applied to the patients. After 2 weeks, except for the tape, the blank patch, hyaluronic acid microneedle and hollow needle were provided to the patients. Except for the hollow needle, the tape, blank patch, and hyaluronic acid microneedle were applied to normal subjects.

The amount of quantified protein can be seen in the graph of FIG. 12 (normal subjects are not included in the IRB). In the graph of FIG. 12, the NET OD (absorbance) value, which is the y-axis, was obtained using the following equation: 'OD value of protein obtained through skin sample' -'OD value of blank state of each patch'. From the protein quantification results, it was confirmed that almost no protein was extracted from the blank patch or hollow needle patch compared to the tape or hyaluronic acid microneedle patch. Thereby, it was confirmed that the blank patch or the hollow needle was not suitable for target factor detection using a non-invasive method.

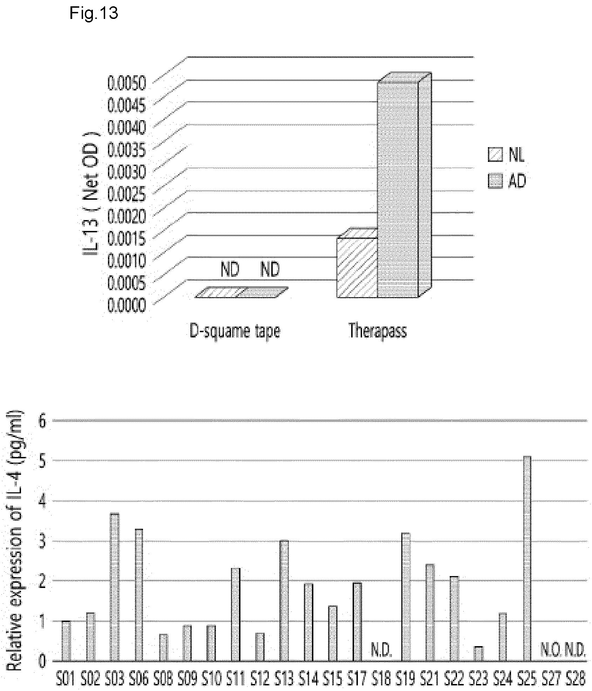

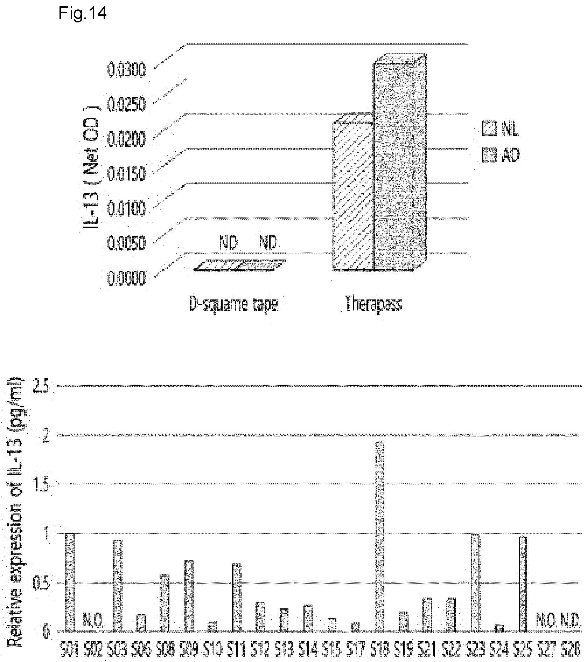

The expression levels of IL-4, IL-13 and IFN-gamma in the samples obtained from normal subjects and atopic dermatitis patients by tissue biopsy, the tape and microneedle were analyzed. In the case of tissue biopsy, RNA was isolated and the expression levels of IL-4, IL-13 and IFN-gamma were analyzed by qRT-PCR assay, and expression of the above targets in the samples from the normal subjects was not identified, unlike the samples from the patients with atopic dermatitis. Next, in the case of the tape and the microneedle, protein was isolated and the expression levels of IL-13 and IFN-gamma were analyzed by ELISA assay. As shown in FIGS. 13 and 14, the expression of the above targets was not identified in the samples obtained from both the normal subjects and the atopic dermatitis patients by the tape, but it was confirmed that the expression levels of IL-13 and IFN-gamma in the samples obtained using the microneedle patch were higher in the atopic dermatitis patients than in the normal subjects.

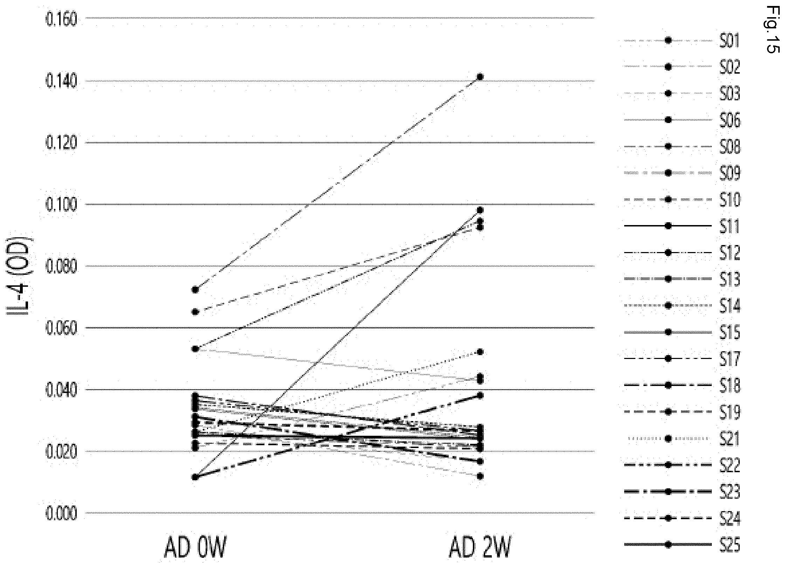

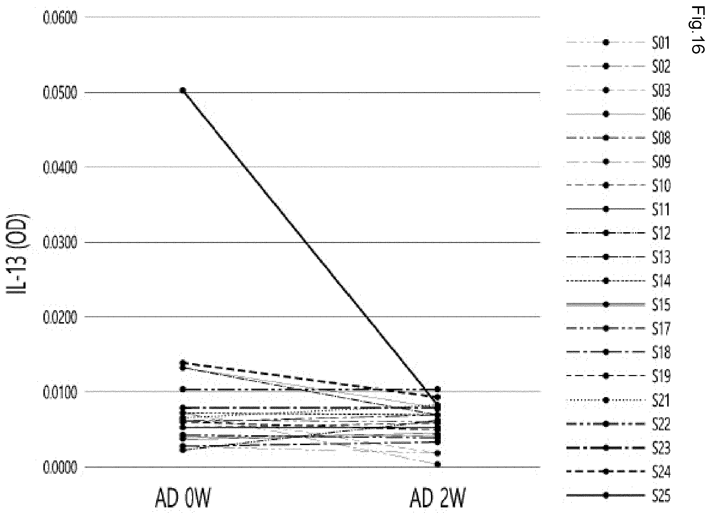

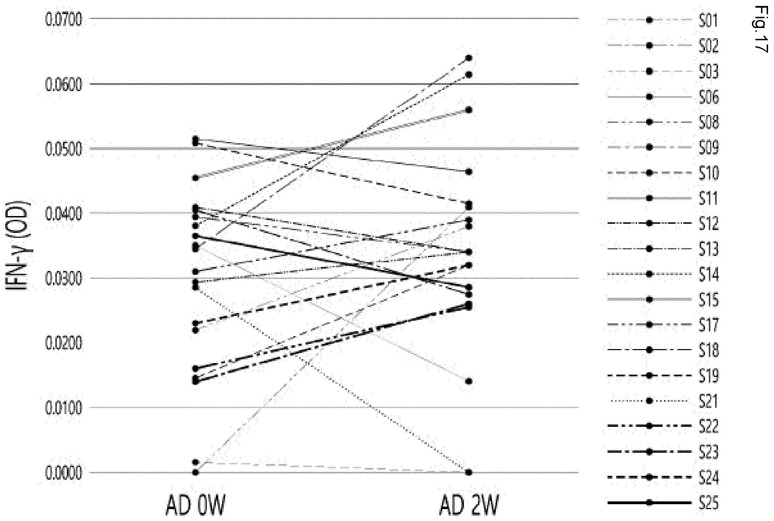

The expression of IL-4, IL-13 and IFN-gamma in the obtained samples was measured by ELISA assay. The results are shown in FIGS. 15 to 17. FIG. 15 is a table showing the results of ELISA assay performed to measure the expression of IL-4 in the skin samples obtained from the clinical trial subjects by the HA microneedle. FIG. 16 is a table showing the results of ELISA assay performed to measure the expression of IL-13 in the skin samples obtained from the clinical trial subjects by the HA microneedle. FIG. 17 is a table showing the results of ELISA assay performed to measure the expression of IFN-gamma in the skin samples obtained from the clinical trial subjects by the HA microneedle.

From the measurement results in FIGS. 15 to 17, it was confirmed that the amounts of the target proteins in the skin samples obtained from the atopic dermatitis patients by the hyaluronic acid microneedle patch upon visit at weeks 0 and 2 changed.

More specifically, the measurement result in FIG. 15 will be described below.

For patient No. 1, the OD value of IL-4 at week 0 is 0.021, and the OD value of IL-4 at week 2 is 0.044.

For patient No. 2, the OD value of IL-4 at week 0 is 0.029, and the OD value of IL-4 at week 2 is 0.012.

For patient No. 2, the OD value of IL-4 at week 0 is 0.026, and the OD value of IL-4 at week 2 is 0.017.

For patient No. 6, the OD value of IL-4 at week 0 is 0.053, and the OD value of IL-4 at week 2 is 0.043.

For patient No. 8, the OD value of IL-4 at week 0 is 0.025, and the OD value of IL-4 at week 2 is 0.021.

For patient No. 9, the OD value of IL-4 at week 0 is 0.072, and the OD value of IL-4 at week 2 is 0.141.

For patient No. 10, the OD value of IL-4 at week 0 is 0.065, and the OD value of IL-4 at week 2 is 0.093.

For patient No. 11, the OD value of IL-4 at week 0 is 0.012, and the OD value of IL-4 at week 2 is 0.098.

For patient No. 12, the OD value of IL-4 at week 0 is 0.053, and the OD value of IL-4 at week 2 is 0.095.

For patient No. 13, the OD value of IL-4 at week 0 is 0.026, and the OD value of IL-4 at week 2 is 0.022.

For patient No. 14, the OD value of IL-4 at week 0 is 0.029, and the OD value of IL-4 at week 2 is 0.027.

For patient No. 15, the OD value of IL-4 at week 0 is 0.034, and the OD value of IL-4 at week 2 is 0.025.

For patient No. 17, the OD value of IL-4 at week 0 is 0.036, and the OD value of IL-4 at week 2 is 0.027.

For patient No. 18, the OD value of IL-4 at week 0 is 0.038, and the OD value of IL-4 at week 2 is 0.025.

For patient No. 19, the OD value of IL-4 at week 0 is 0.023, and the OD value of IL-4 at week 2 is 0.021.

For patient No. 21, the OD value of IL-4 at week 0 is 0.026, and the OD value of IL-4 at week 2 is 0.052.

For patient No. 22, the OD value of IL-4 at week 0 is 0.012, and the OD value of IL-4 at week 2 is 0.038.

For patient No. 23, the OD value of IL-4 at week 0 is 0.031, and the OD value of IL-4 at week 2 is 0.017.

For patient No. 24, the OD value of IL-4 at week 0 is 0.035, and the OD value of IL-4 at week 2 is 0.028.

For patient No. 25, the OD value of IL-4 at week 0 is 0.025, and the OD value of IL-4 at week 2 is 0.024.

More specifically, the measurement results in FIG. 16 will be described below.

For patient No. 1, the net OD value of IL-13 at week 0 is 0.0027, and the net OD value of IL-4 at week 2 is 0.0017.

For patient No. 2, the net OD value of IL-13 at week 0 is 0.0072, and the net OD value of IL-4 at week 2 is 0.0002.

For patient No. 3, the net OD value of IL-13 at week 0 is 0.0072, and the net OD value of IL-4 at week 2 is 0.0017.

For patient No. 6, the net OD value of IL-13 at week 0 is 0.0132, and the net OD value of IL-4 at week 2 is 0.0077.

For patient No. 8, the net OD value of IL-13 at week 0 is 0.0062, and the net OD value of IL-4 at week 2 is 0.0059.

For patient No. 9, the net OD value of IL-13 at week 0 is 0.0059, and the net OD value of IL-4 at week 2 is 0.0069.

For patient No. 10, the net OD value of IL-13 at week 0 is 0.0052, and the net OD value of IL-4 at week 2 is 0.0059.

For patient No. 11, the net OD value of IL-13 at week 0 is 0.0052, and the net OD value of IL-4 at week 2 is 0.0052.

For patient No. 12, the net OD value of IL-13 at week 0 is 0.0022, and the net OD value of IL-4 at week 2 is 0.0062.

For patient No. 13, the net OD value of IL-13 at week 0 is 0.0132, and the net OD value of IL-4 at week 2 is 0.0067.

For patient No. 14, the net OD value of IL-13 at week 0 is 0.0072, and the net OD value of IL-4 at week 2 is 0.0069.

For patient No. 15, the net OD value of IL-13 at week 0 is 0.0037, and the net OD value of IL-4 at week 2 is 0.0042.

For patient No. 17, the net OD value of IL-13 at week 0 is 0.0042, and the net OD value of IL-4 at week 2 is 0.0037.

For patient No. 18, the net OD value of IL-13 at week 0 is 0.0027, and the net OD value of IL-4 at week 2 is 0.0032.

For patient No. 19, the net OD value of IL-13 at week 0 is 0.0059, and the net OD value of IL-4 at week 2 is 0.0049.

For patient No. 21, the net OD value of IL-13 at week 0 is 0.0065, and the net OD value of IL-4 at week 2 is 0.0082.

For patient No. 22, the net OD value of IL-13 at week 0 is 0.0102, and the net OD value of IL-4 at week 2 is 0.0102.

For patient No. 23, the net OD value of IL-13 at week 0 is 0.0079, and the net OD value of IL-4 at week 2 is 0.0079.

For patient No. 24, the net OD value of IL-13 at week 0 is 0.0137, and the net OD value of IL-4 at week 2 is 0.0092.

For patient No. 25, the net OD value of IL-13 at week 0 is 0.0502, and the net OD value of IL-4 at week 2 is 0.0082.

More specifically, the measurement results in FIG. 17 will be described below.

For patient No. 1, the net OD value of IFN-gamma at week 0 is 0.0220, and the net OD value of IFN-gamma at week 2 is 0.0380.

For patient No. 2, the net OD value of IFN-gamma at week 0 is -0.0005, and the net OD value of IFN-gamma at week 2 is 0.0410.

For patient No. 3, the net OD value of IFN-gamma at week 0 is 0.0015, and the net OD value of IFN-gamma at week 2 is -0.0045.

For patient No. 6, the net OD value of IFN-gamma at week 0 is 0.0350, and the net OD value of IFN-gamma at week 2 is 0.0140.

For patient No. 8, the net OD value of IFN-gamma at week 0 is 0.0395, and the net OD value of IFN-gamma at week 2 is 0.0340.

For patient No. 9, the net OD value of IFN-gamma at week 0 is 0.0345, and the net OD value of IFN-gamma at week 2 is 0.0640.

For patient No. 10, the net OD value of IFN-gamma at week 0 is 0.0145, and the net OD value of IFN-gamma at week 2 is 0.0320.

For patient No. 11, the net OD value of IFN-gamma at week 0 is 0.0515, and the net OD value of IFN-gamma at week 2 is 0.0465.

For patient No. 12, the net OD value of IFN-gamma at week 0 is 0.0295, and the net OD value of IFN-gamma at week 2 is 0.0340.

For patient No. 13, the net OD value of IFN-gamma at week 0 is 0.0410, and the net OD value of IFN-gamma at week 2 is 0.0340.

For patient No. 14, the net OD value of IFN-gamma at week 0 is 0.0380, and the net OD value of IFN-gamma at week 2 is 0.0615.

For patient No. 15, the net OD value of IFN-gamma at week 0 is 0.0455, and the net OD value of IFN-gamma at week 2 is 0.0560.

For patient No. 17, the net OD value of IFN-gamma at week 0 is 0.0310, and the net OD value of IFN-gamma at week 2 is 0.0390.

For patient No. 18, the net OD value of IFN-gamma at week 0 is 0.0405, and the net OD value of IFN-gamma at week 2 is 0.0275.

For patient No. 19, the net OD value of IFN-gamma at week 0 is 0.0510, and the net OD value of IFN-gamma at week 2 is 0.0415.

For patient No. 21, the net OD value of IFN-gamma at week 0 is 0.0285, and the net OD value of IFN-gamma at week 2 is 0.0000.

For patient No. 22, the net OD value of IFN-gamma at week 0 is 0.0160, and the net OD value of IFN-gamma at week 2 is 0.0255.

For patient No. 23, the net OD value of IFN-gamma at week 0 is 0.0140, and the net OD value of IFN-gamma at week 2 is 0.0260.

For patient No. 24, the net OD value of IFN-gamma at week 0 is 0.0230, and the net OD value of IFN-gamma at week 2 is 0.0320.

For patient No. 24, the net OD value of IFN-gamma at week 0 is 0.0365, and the net OD value of IFN-gamma at week 2 is 0.0285.

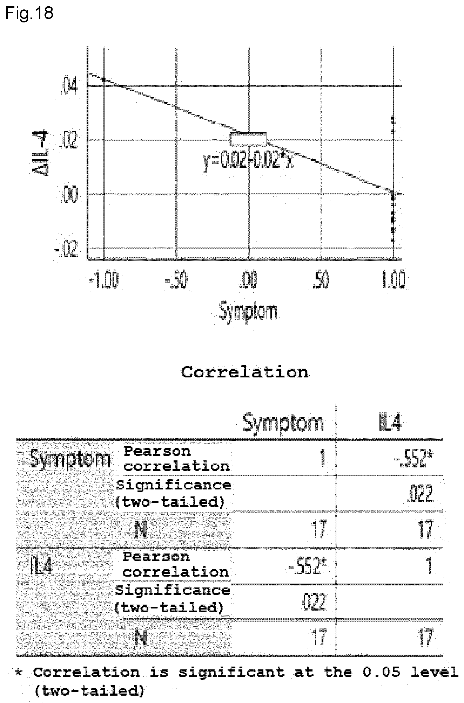

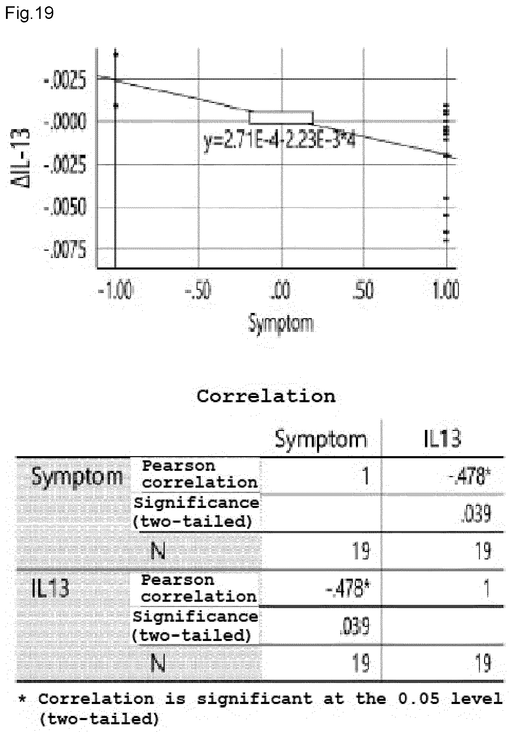

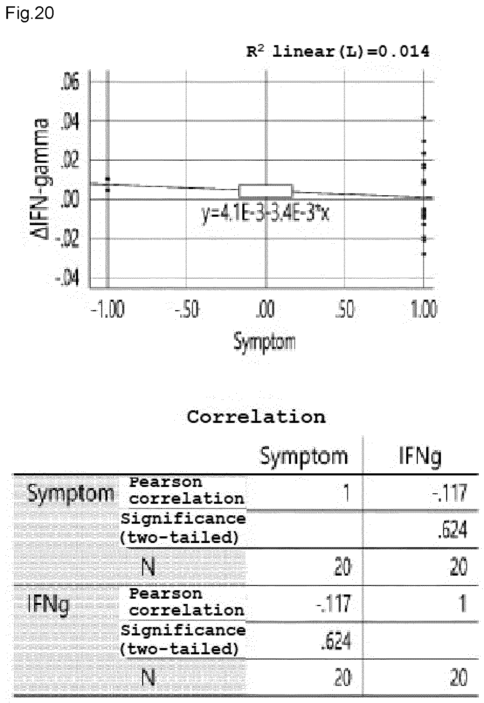

The present inventors analyzed the correlation between the expression of target proteins in the skin samples, obtained by the hyaluronic acid microneedle patch, and the clinical course of atopic dermatitis by statistical analysis (SPSS). The results are shown in FIGS. 18 to 20.

For statistical analysis of the correlation, the present inventors assigned different scores according to the clinical course of the lesion. A score of -1 was assigned to patient Nos. 12 and 15, who were clinically judged to have significantly aggravated or slightly aggravated symptoms, and a score of 1 was assigned to all patients other than patent No. 15. Referring to the clinical course after 2 weeks shown on the rightmost side of the table in FIG. 11, in the case of patient No. 12 marked as "so so + urticaria" symptoms, a clinical judgment was made that the symptoms were slightly aggravated. In the case of patient No. 15 marked as "aggravated", a clinical judgment was made that the symptoms were significantly aggravated. For the remaining patients, a clinical judgment was made that the symptoms were maintained or improved.

The x-axis of the graph shown in the top of each of FIGS. 18 to 20 represents the clinical course. The y-axes of these graphs represent changes in IL-4, IL-13, and IFN-gamma ('expression level of target at 2 weeks - 'expression level of target at week 0'), respectively. As a result of statistical analysis, it was confirmed that there was a significant correlation between the clinical course of atopic dermatitis and changes in IL-4 and IL-13 (p<0.05). In addition, as a result of statistical analysis, it was confirmed that there was no correlation between the clinical course of atopic dermatitis and a change in IFN-gamma. Thereby, the clinical usefulness of target factor detection using a non-invasive method (application of the hyaluronic acid microneedle patch) was verified. In summary, it was confirmed that the detected amounts of interleukin-4 and interleukin-13 decreased in the atopic dermatitis patients having improved atopic symptoms, and that the detected amounts of interleukin-4 and interleukin-13 increased in the patients having aggravated atopic symptoms. Accordingly, reliable measurement of the activity of atopic dermatitis also became possible without intervention of the clinical judgment of medical staff, including doctors, by attaching the biodegradable high-molecular-weight hyaluronic acid microneedle patch to the human body without invasive procedures such as tissue biopsy, detaching the microneedle patch, and then measuring changes in the expression levels of important biomarkers involved in pathogenesis.

In addition, using the minimally invasive kit for diagnosing atopic dermatitis according to one embodiment of the present invention, which consists of a microneedle patch formed of biodegradable polymer hyaluronic acid and a device capable of quantitative analysis of proteins obtained from human skin, it is possible to easily and accurately evaluate the activity of atopic dermatitis by simply comparing the values of the amounts of interleukin-4 and interleukin-13, obtained by the quantitative analysis device, with predetermined values without depending on the medical knowledge or experience of the medical personnel. The above-described evaluation of the activity of atopic dermatitis may also be automatically performed by a functional unit that may be included in the kit for diagnosing atopic dermatitis according to one embodiment of the present invention. In this case, the functional unit may be configured to include: a storage unit configured to store data on the amounts of interleukin-4 and interleukin-13 obtained from the quantitative analysis device and data on the correlation between these amounts and atopic dermatitis activity; an interface unit configured to transfer data from the quantitative analysis device; a comparison determination unit configured to compare and determine the data stored in the storage unit and the data transferred from the quantitative analysis device, thereby generating information on atopic dermatitis activity; and a display unit configured to display the information generated by the comparison determination unit.

Although the present invention has been described in detail described using specific matters such as specific components, limited embodiments, and the accompanying drawings, these embodiments are provided only for assisting in the entire understanding of the present invention, and the present invention is not limited to these embodiments. Various modifications and changes may be made by those skilled in the art to which the present invention pertains from this description.

Therefore, the spirit of the present invention should not be construed as being limited to the above-described embodiments, and the following claims as well as all modified equally or equivalently to the claims are intended to fall within the scopes and spirit of the present invention.

## Claims

1. A minimally invasive method for diagnosing atopic dermatitis comprising steps of:
applying, to a skin of a subject, a microneedle patch comprising a plurality of microneedles, which are formed of biodegradable polymer hyaluronic acid and have a solid structure, and a bottom layer on which the plurality of microneedles are formed;
maintaining the microneedle patch for a predetermined time in a state in which the microneedle patch is attached to the skin of the subject;
separating the microneedle patch from the skin of the subject after the predetermined time and putting the proteins from the subject, adsorbed onto surfaces of the microneedles of the microneedle patch, into quantitative testing;
reading amounts of interleukin-4 and interleukin-13, adsorbed onto surfaces of the microneedles of the microneedle patch, in the quantitative testing; and evaluating an activity of atopic dermatitis based on the read amounts of interleukin-4 and interleukin-13.

2. The minimally invasive method for diagnosing atopic dermatitis according to claim 1, wherein the quantitative testing is ELISA measurement.

3. The minimally invasive method for diagnosing atopic dermatitis according to claim 2, wherein an amount of detected IFN-gamma is not considered in evaluating the activity of atopic dermatitis.

4. The minimally invasive method for diagnosing atopic dermatitis according to claim 3, wherein the predetermined time is predetermined in consideration of a molecular weight-dependent dissolution rate of the hyaluronic acid constituting the microneedles.

5. The minimally invasive method for diagnosing atopic dermatitis according to claim 4, wherein, as the molecular weight of the hyaluronic acid constituting the microneedles increases from a low molecular weight to a high molecular weight, the time of attachment to the skin of the subject is lengthened and biodetection performance is improved.

6. A minimally invasive kit for diagnosing atopic dermatitis comprising: a device configured to quantitatively analyze amounts of proteins extracted from an skin of a subject; and
a microneedle patch comprising a plurality of microneedles, which are formed of biodegradable polymer hyaluronic acid and have a solid structure, and a bottom layer on which the plurality of microneedles are formed,
wherein the microneedle patch is applied to the skin of the subject, maintained for a predetermined time, and then separated from the skin, and in the separated state, the proteins from the subject, adsorbed onto surfaces of the microneedles of the microneedle patch, are quantitatively analyzed by the device, the device reads amounts of interleukin-4 and interleukin-13 adsorbed onto the surfaces of the microneedles of the microneedle patch, and an activity of atopic dermatitis is evaluated based on the read amounts of interleukin-4 and interleukin-13.

7. The minimally invasive kit for diagnosing atopic dermatitis according to claim 6, wherein ELISA measurement is performed by the device.

8. The minimally invasive kit for diagnosing atopic dermatitis according to claim 7, wherein an amount of detected IFN-gamma is not considered in evaluating the activity of atopic dermatitis.

9. The minimally invasive kit for diagnosing atopic dermatitis according to claim 8, wherein the predetermined time is predetermined in consideration of a molecular weight-dependent dissolution rate of the hyaluronic acid constituting the microneedles.

10. The minimally invasive kit for diagnosing atopic dermatitis according to claim 9, wherein, as the molecular weight of the hyaluronic acid constituting the microneedles increases from a low molecular weight to a high molecular weight, the time of attachment to the skin of the subject is lengthened and biodetection performance is improved.

## Patentansprüche

1. Minimal-invasives Verfahren zum Diagnostizieren atopischer Dermatitis umfassend die Schritte:
Aufbringen, auf die Haut einer Person, eines Mikronadelpflasters umfassend eine Vielzahl von Mikronadeln, die aus biologisch abbaubarer polymerer Hyaluronsäure gebildet sind und eine feste Struktur aufweisen, und eine untere Schicht, auf der die Vielzahl von Mikronadeln gebildet sind;
Aufrechterhalten des Mikronadelpflasters für eine vorgegebene Zeit in einem Zustand, in dem das Mikronadelpflaster auf der Haut der Person befestigt ist;
Trennen des Mikronadelpflasters von der Haut der Person nach der vorgegebenen Zeit und Unterziehen der Proteine von der Person, die auf die Oberflächen der Mikronadeln des Mikronadelpflasters adsorbiert sind, einem quantitativen Test;
Ablesen der Mengen an Interleukin-4 und Interleukin-13, die auf die Oberflächen der Mikronadeln des Mikronadelpflasters adsorbiert sind, in dem quantitativen Test; und Evaluieren der Aktivität der atopischen Dermatitis basierend auf den abgelesenen Mengen an Interleukin-4 und Interleukin-13.

2. Minimal-invasives Verfahren zum Diagnostizieren atopischer Dermatitis nach Anspruch 1, wobei der quantitative Test eine ELISA-Messung ist.

3. Minimal-invasives Verfahren zum Diagnostizieren atopischer Dermatitis nach Anspruch 2, wobei die Menge an detektiertem IFN-gamma beim Evaluieren der Aktivität der atopischen Dermatitis nicht in Betracht gezogen wird.

4. Minimal-invasives Verfahren zum Diagnostizieren atopischer Dermatitis nach Anspruch 3, wobei die vorgegebene Zeit unter Berücksichtigung der molekulargewichtsabhängigen Auflösungsgeschwindigkeit der die Mikronadeln bildenden Hyaluronsäure im Voraus festgelegt wird.

5. Minimal-invasives Verfahren zum Diagnostizieren atopischer Dermatitis nach Anspruch 4, wobei, wenn das Molekulargewicht der die Mikronadeln bildenden Hyaluronsäure von einem niedrigen Molekulargewicht auf ein hohes Molekulargewicht zunimmt, die Zeit der Befestigung auf der Haut der Person verlängert wird und die Biodetektionsperformance verbessert wird.

6. Minimal-invasives Kit zum Diagnostizieren atopischer Dermatitis umfassend: eine Vorrichtung, die dazu konfiguriert ist, aus der Haut einer Person extrahierte Mengen von Proteinen quantitativ zu analysieren; und
ein Mikronadelpflaster umfassend eine Vielzahl von Mikronadeln, die aus biologisch abbaubarer polymerer Hyaluronsäure gebildet sind und eine feste Struktur aufweisen, und eine untere Schicht, auf der die Vielzahl von Mikronadeln gebildet sind;
wobei das Mikronadelpflaster auf die Haut der Person aufgebracht wird, für eine vorgegebene Zeit dort verbleibt, und dann von der Haut getrennt wird, und in dem getrennten Zustand die Proteine von der Person, die auf die Oberflächen der Mikronadeln des Mikronadelpflasters adsorbiert sind, durch die Vorrichtung quantitativ analysiert werden, die Vorrichtung die Mengen an Interleukin-4 und Interleukin-13, die auf die Oberflächen der Mikronadeln des Mikronadelpflasters adsorbiert sind, abliest und die Aktivität der atopischen Dermatitis basierend auf den abgelesenen Mengen an Interleukin-4 und Interleukin-13 evaluiert wird.

7. Minimal-invasives Kit zum Diagnostizieren atopischer Dermatitis nach Anspruch 6, wobei durch die Vorrichtung eine ELISA-Messung durchgeführt wird.

8. Minimal-invasives Kit zum Diagnostizieren atopischer Dermatitis nach Anspruch 7, wobei die Menge an detektiertem IFN-gamma beim Evaluieren der Aktivität der atopischen Dermatitis nicht in Betracht gezogen wird.

9. Minimal-invasives Kit zum Diagnostizieren atopischer Dermatitis nach Anspruch 8, wobei die vorgegebene Zeit unter Berücksichtigung der molekulargewichtsabhängigen Auflösungsgeschwindigkeit der die Mikronadeln bildenden Hyaluronsäure im Voraus festgelegt wird.

10. Minimal-invasives Kit zum Diagnostizieren atopischer Dermatitis nach Anspruch 9, wobei, wenn das Molekulargewicht der die Mikronadeln bildenden Hyaluronsäure von einem niedrigen Molekulargewicht auf ein hohes Molekulargewicht zunimmt, die Zeit der Befestigung auf der Haut der Person verlängert wird und die Biodetektionsperformance verbessert wird.

## Revendications

1. Procédé minimalement invasif pour le diagnostic de la dermatite atopique, comprenant les étapes consistant à :
appliquer, sur la peau d'un sujet, un timbre transdermique à micro-aiguilles comprenant une pluralité de micro-aiguilles, lesquelles sont formées d'un polymère biodégradable d'acide hyaluronique et présentent une structure solide, ainsi qu'une couche inférieure sur laquelle la pluralité de micro-aiguilles est formée ;
maintenir le timbre transdermique à micro-aiguilles pendant une durée prédéterminée dans un état dans lequel le timbre transdermique à micro-aiguilles est fixé à la peau du sujet ;
séparer le timbre transdermique à micro-aiguilles de la peau du sujet après la durée prédéterminée, et soumettre des protéines provenant du sujet, adsorbées à la surface des micro-aiguilles du timbre transdermique à micro-aiguilles, à un test quantitatif ;
lire les quantités d'interleukine-4 et d'interleukine-13 adsorbées à la surface des micro-aiguilles du timbre transdermique à micro-aiguilles lors du test quantitatif ; et
évaluer une activité de dermatite atopique sur la base des quantités d'interleukine-4 et d'interleukine-13 lues.

2. Procédé minimalement invasif pour le diagnostic de la dermatite atopique selon la revendication 1, dans lequel le test quantitatif est une mesure par ELISA.

3. Procédé minimalement invasif pour le diagnostic de la dermatite atopique selon la revendication 2, dans lequel une quantité d'IFN-gamma détectée n'est pas prise en compte dans l'évaluation de l'activité de la dermatite atopique

4. Procédé minimalement invasif pour le diagnostic de la dermatite atopique selon la revendication 3, dans lequel la durée prédéterminée est définie en fonction d'un taux de dissolution dépendant du poids moléculaire de l'acide hyaluronique constituant les micro-aiguilles.

5. Procédé minimalement invasif pour le diagnostic de la dermatite atopique selon la revendication 4, dans lequel, lorsque le poids moléculaire de l'acide hyaluronique constituant les micro-aiguilles augmente d'un faible poids moléculaire à un poids moléculaire élevé, la durée de fixation à la peau du sujet est allongée et la performance de biodétection est améliorée.

6. Kit minimalement invasif pour le diagnostic de la dermatite atopique, comprenant : un dispositif configuré pour analyser quantitativement des quantités de protéines extraites de la peau d'un sujet ; et
un timbre transdermique à micro-aiguilles comprenant une pluralité de micro-aiguilles, lesquelles sont formées d'un polymère biodégradable d'acide hyaluronique et présentent une structure solide, ainsi qu'une couche inférieure sur laquelle la pluralité de micro-aiguilles est formée,
dans lequel le timbre transdermique à micro-aiguilles est appliqué sur la peau du sujet, maintenu pendant une durée prédéterminée, puis séparé de la peau, et, dans l'état séparé, les protéines provenant du sujet, adsorbées à la surface des micro-aiguilles du timbre transdermique à micro-aiguilles, sont analysées quantitativement par le dispositif, ledit dispositif lisant les quantités d'interleukine-4 et d'interleukine-13 adsorbées à la surface des micro-aiguilles du timbre transdermique à micro-aiguilles, et une activité de dermatite atopique étant évaluée sur la base des quantités d'interleukine-4 et d'interleukine-13 ainsi lues.

7. Kit minimalement invasif pour le diagnostic de la dermatite atopique selon la revendication 6, dans lequel une mesure par ELISA est effectuée par le dispositif.

8. Kit minimalement invasif pour le diagnostic de la dermatite atopique selon la revendication 7, dans lequel une quantité d'IFN-gamma détectée n'est pas prise en compte dans l'évaluation de l'activité de la dermatite atopique.

9. Kit minimalement invasif pour le diagnostic de la dermatite atopique selon la revendication 8, dans lequel la durée prédéterminée est définie en fonction d'un taux de dissolution dépendant du poids moléculaire de l'acide hyaluronique constituant les micro-aiguilles.

10. Kit minimalement invasif pour le diagnostic de la dermatite atopique selon la revendication 9, dans lequel, lorsque le poids moléculaire de l'acide hyaluronique constituant les micro-aiguilles augmente d'un faible poids moléculaire à un poids moléculaire élevé, la durée de fixation à la peau du sujet est allongée et la performance de biodétection est améliorée.
